# FASCICULE DE BREVET EUROPEEN

(11) **EP 2 149 363 B1**
(45) Date de publication et mention de la délivrance du brevet: **06.01.2016**
(21) Numéro de dépôt: 09151393.7
(22) Date de dépôt: 27.01.2009
(51) Int. Cl.: A61K 8/31, A61Q 5/06, A61K 8/81, A61Q 5/12, A61K 8/06

(54) **Composition cosmétique aqueuse comprenant un copolymère vinylformamide / vinylamine, un corps gras non siliconé, un tensioactif et une silicone.**
Wässrige kosmetische Zusammensetzung enthaltend ein Copolymer aus Vinylformamid/Vinylformamin, ein nicht silikoniertes Fettderivat und eine Tenside
Aqueous cosmetic composition comprising a copolymer of vinylformamide / vinylformamine, a non silicon fatty derivative and a surfactant

(30) Priorité: 31.01.2008 FR 0850631
(43) Date de publication de la demande: 03.02.2010
(73) Titulaire: L'Oréal, 75008 Paris (FR)
(72) Inventeur: Benabdillah, Katarina, 30140 Saint Jean du Pin (FR); Mathonneau, Estelle, 75010, PARIS (FR)
(74) Mandataire: Martin-Charbonneau, Virginie

(56) Documents cités:
- EP-A- 1 779 894
- WO-A-2007/003784
- DE-A1-102005 014 293
- JP-A- 2002 255 756
- US-A- 5 632 977
- US-A1- 2006 286 057
- US-A1- 2007 110 690

## Description

La présente invention est relative à une composition cosmétique aqueuse comprenant, dans un milieu cosmétiquement acceptable, un ou plusieurs corps gras non siliconés, un ou plusieurs tensioactifs, un ou plusieurs copolymères vinylformamide / vinylamine et une ou plusieurs silicones.

La présente invention concerne également une utilisation de cette composition pour le traitement cosmétique capillaire, et en particulier pour le coiffage et le soin des cheveux, ainsi qu'un procédé de traitement cosmétique des cheveux mettant en oeuvre une telle composition.

La présente invention concerne enfin un procédé de fixation et/ou de mise en forme des cheveux, mettant en oeuvre une composition cosmétique aqueuse comprenant, dans un milieu cosmétiquement acceptable, un ou plusieurs corps gras non siliconés, un ou plusieurs tensioactifs, et un ou plusieurs copolymères vinylformamide / vinylamine.

Les produits de coiffage sont habituellement utilisés pour construire, structurer la coiffure et lui apporter une tenue durable. Ils se présentent usuellement sous forme de lotions, gels, mousses, crèmes, sprays, etc. Les compositions correspondantes comprennent généralement un ou plusieurs polymères filmogènes ou « polymères fixants », dans un milieu cosmétiquement acceptable. Ces polymères permettent la formation d'un film gainant sur les cheveux, assurant ainsi le maintien de la coiffure.

Toutefois, les films de polymère fixant ainsi formés présentent l'inconvénient d'être relativement friables, ce qui limite dans le temps la tenue de la coiffure, et engendre la formation sur les cheveux de résidus inesthétiques.

Ainsi, les produits de coiffage classiques conduisent à une fixation de la coiffure et des effets coiffants qui s'estompent progressivement au cours du temps. En particulier, lorsque le produit est appliqué le matin, les effets coiffants s'estompent au fur et à mesure dans la journée. Le lendemain, les effets coiffants sont faibles, voire inexistants.

Ce problème est d'autant plus important lorsqu'il s'agit de coiffer et mettre en forme des chevelures frisées, ou des chevelures dites « ethniques », c'est-à-dire des cheveux de type par exemple brésilien, africain, maghrébin, lesquels présentent la particularité d'être généralement secs et frisés, et particulièrement difficiles à mettre en forme.

Pour remédier à ce problème, il est connu d'incorporer dans les produits de coiffage des polymères à très haut pouvoir fixant, et/ou d'augmenter la concentration en polymère fixant. Toutefois, l'emploi de tels produits extrêmement fixants entraine un certain nombre d'inconvénients. En particulier, ces produits ne permettent généralement pas d'avoir une cohésion suffisante des mèches entre elles, conduisent à un toucher sec et rugueux des cheveux et sont difficiles à éliminer au shampooing.

Il existe donc un besoin pour des compositions capillaires permettant d'obtenir une fixation forte, avec une cohésion suffisante des mèches (pas de désolidarisation en brindilles), et une fixation durable de la coiffure, avec des effets coiffants persistant toute la journée voire plusieurs jours, tout en s'éliminant facilement au shampooing et en procurant un toucher cosmétique agréable.

La demande de brevet internationale WO 96/03969 décrit d'une manière générale des compositions cosmétiques destinées à la fixation et/ou au conditionnement des cheveux, qui comprennent un homopolymère de vinylformamide ou un copolymère de vinylformamide et d'un ou plusieurs autres monomères vinyliques, en association avec au moins un ingrédient choisi parmi les agents conditionnants, les agents émulsifiants, les tensioactifs, les modificateurs de viscosité, les agents gélifiants, les agents opacifiants, les agents stabilisants, les conservateurs, les agents séquestrants, les agents chélatants, les agents nacrants, les agents clarifiants, les parfums, les colorants, les agents propulseurs, les solvants organiques et l'eau.

Par ailleurs, le brevet américain US 4 421 602 décrit des copolymères vinylformamide / vinylamine, leur préparation et leur utilisation dans l'industrie papetière pour améliorer la rétention, le taux d'écoulement, et la floculation.

La demande de brevet JP 2002 255756 décrit une composition de mise en forme pour les cheveux, qui comprend de 0,01% à 10% en poids d'un copolymère à base de monomères alcool vinylique et amine vinylique, un composé pulvérulent et un acide, et qui présente un pH allant de 4 à 9.

La demande de brevet US 2006/0286057 décrit une composition aqueuse de support pour des composés insolubles dans l'eau, qui contient : au moins une polyamine possédant au moins deux groupements amino, au moins un tensioactif non ionique, au moins une silicone anionique et au moins un composé insoluble dans l'eau.

La demande de brevet US 2007/0110690 décrit un procédé pour empêcher les cheveux de friser, consistant à appliquer sur les cheveux une composition contenant au moins une polyamine possédant au moins deux groupements amino, au moins une silicone anionique, en option, au moins un tensioactif et, également en option, au moins un agent filmogène.

Le brevet US 5,632,977 décrit des compositions de soin capillaire contenant un polymère préparé à partir de monomères vinylformamide, qui peut être un homopolymère ou un copolymère contenant également d'autres monomères vinyliques.

La demande de brevet DE 10 2005 014 293 décrit un agent épaississant constitué d'un polymère ou d'un mélange de polymères différents, qui comportent des groupements de type amide et/ou amino, et l'emploi d'un tel agent dans des compositions aqueuses destinées à la cosmétique, la nutrition, la dermatologie, la pharmacie et la détergence.

La demande de brevet WO 2007/003784 décrit une composition cosmétique ou pharmaceutique contenant un polymère éthylénique cationique lequel comprend au moins une fonction amine primaire, secondaire ou tertiaire protonable, au moins partiellement neutralisée par un acide organique polymérique.

Par ailleurs, dans le domaine de la teinture capillaire, la demande EP 1 779 894 décrit l'utilisation de polymères du type des vinylamines, afin d'améliorer l'intensité de la coloration.

La Demanderesse a maintenant découvert que, de manière surprenante, l'association en milieu aqueux d'un copolymère vinylformamide / vinylamine avec un corps gras non siliconé et un tensioactif permettait d'obtenir une composition cosmétique capillaire apportant des propriétés de coiffage améliorées.

Notamment, une telle association permet d'obtenir des produits de coiffage procurant une fixation forte, avec une cohésion suffisante des mèches (pas de désolidarisation en brindilles), et durable de la coiffure, tout en s'éliminant facilement au lavage et en procurant un toucher cosmétique agréable des cheveux.

La présente invention permet ainsi de préparer des produits de coiffage procurant des degrés de fixation très élevés, une très longue tenue de la coiffure au cours du temps, et une bonne résistance de celle-ci aux sollicitations mécaniques.

La présente invention permet également de préparer des produits de coiffage procurant un toucher des cheveux particulièrement doux et agréable, et des cheveux particulièrement déliés.

Les propriétés d'une telle composition sont améliorées lorsque celle-ci contient en outre une ou plusieurs silicones.

La présente invention a donc pour objet une composition cosmétique comprenant, dans un milieu aqueux cosmétiquement acceptable :
- un ou plusieurs corps gras non siliconés,
- un ou plusieurs tensioactifs,
- un ou plusieurs copolymères vinylformamide / vinylamine comprenant :
   de 20 à 40 % en moles de motifs de formule suivante A : 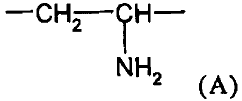
   et de 60 à 80 % en moles de motifs de formule suivante B: 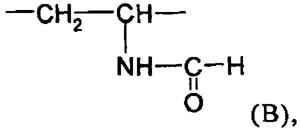 et
- une ou plusieurs silicones.

Les compositions selon l'invention se sont avérées particulièrement appropriées pour le coiffage des cheveux frisés et notamment des cheveux ethniques, par exemple de type brésilien, africain et maghrébin, qui présentent la caractéristique d'être particulièrement difficiles à mettre en forme de manière durable. Ainsi, la composition selon l'invention permet par exemple de diminuer le volume de la chevelure, ou de réaliser des coiffures plaquées de longue durée.

D'autres objets, caractéristiques, aspects et avantages de l'invention apparaîtront encore plus clairement à la lecture de la description et des exemples qui suivent.

Selon un mode de réalisation, la composition selon la présente invention comprend, dans un milieu aqueux cosmétiquement acceptable, un ou plusieurs corps gras non siliconés, un ou plusieurs tensioactifs, un ou plusieurs copolymères vinylformamide / vinylamine, et une ou plusieurs silicones.

Par « milieu cosmétiquement acceptable », on entend un milieu compatible avec les matières kératiniques et en particulier les cheveux.

Par « milieu aqueux », on entend que le milieu cosmétiquement acceptable utilisé dans les compositions selon la présente invention est un milieu contenant de l'eau.

De préférence, la composition selon l'invention comprend au moins 10% en poids d'eau, par rapport au poids total de la composition. De manière plus préférée, la composition selon l'invention comprend au moins 20% en poids d'eau, de manière plus préférée au moins 30% en poids, et de manière encore plus préférée au moins 40 % en poids, par rapport au poids total de la composition.

Le milieu cosmétiquement acceptable peut également comprendre un ou plusieurs solvants organiques cosmétiquement acceptables, qui peuvent être notamment choisis parmi les alcools inférieurs en C₁-C₄, tels que l'éthanol, l'isopropanol, le tertio-butanol ou le n-butanol ; les polyols comme le propylèneglycol et le glycérol ; les éthers de polyols ; les alcanes en C₅-C₁₀ ; les cétones en C₃₋₄ comme l'acétone et la méthyléthylcétone ; les acétates d'alkyle en C₁-C₄ comme l'acétate de méthyle, l'acétate d'éthyle et l'acétate de butyle ; le diméthoxyéthane, le diéthoxyéthane ; et leurs mélanges.

Parmi les solvants préférés, on peut citer le glycérol et l'éthanol.

Le ou les copolymères vinylformamide / vinylamine utilisés dans les compositions selon l'invention comprennent de 20 à 40% en moles de motifs de formule A. Ils comprennent également de 60 à 80% en moles de motifs de formule B.

Les copolymères selon l'invention peuvent être obtenus par exemple par hydrolyse partielle de polyvinylformamide. Cette hydrolyse peut se faire en milieu acide ou basique.

Le ou les copolymères vinylformamide / vinylamine selon l'invention peuvent éventuellement comprendre un ou plusieurs motifs monomères additionnels. Dans ce cas, ces derniers représentent de préférence moins de 20% en moles du copolymère.

Selon un mode de réalisation préféré, le ou les copolymères vinylformamide / vinylamine selon l'invention sont constitués uniquement de motifs de formule A et de motifs de formule B.

La masse moléculaire moyenne en poids du copolymère, mesurée par diffraction de la lumière, peut varier 10.000 à 30.000.000 g/mole, de préférence de 40.000 à 1.000.000 et plus particulièrement de 100.000 à 500.000 g/mole.

La densité de charge cationique du copolymère vinylformamide / vinylamine peut varier de 2 meq/g à 20 meq/g, de préférence de 2,5 à 15 et plus particulièrement de 3,5 à 10 meq/g.

A titre d'exemple de copolymères vinylformamide / vinylamine utilisables dans les compositions selon l'invention, citons entre autres les produits commercialisés sous la dénomination LUPAMIN par la société BASF, tels que par exemple, et de manière non limitative, les produits proposés sous la dénomination LUPAMIN 9030 et LUPAMIN 9010.

Le ou les copolymères vinylformamide / vinylamine sont présents dans les compositions selon l'invention dans des proportions allant, de préférence, de 0,05 à 25 % en poids, plus préférentiellement de 0,1 à 20 % en poids et plus particulièrement de 0,5 à 10 % en poids, par rapport au poids total de la composition.

Par corps gras, on entend au sens de la présente invention un composé organique qui, à température ambiante (25°C) et à pression atmosphérique, est insoluble dans l'eau (c'est-à-dire, présente une solubilité dans l'eau inférieure à 1% en poids et de préférence inférieure à 0,5% en poids), et est soluble, dans les mêmes conditions de température et de pression, dans au moins un solvant organique (par exemple l'éthanol, le chloroforme, ou le benzène) à au moins 1% en poids.

Les corps gras non siliconés utilisables dans les compositions selon la présente invention sont notamment toutes les huiles, cires, résines non siliconées organiques ou minérales, naturelles ou synthétiques répondant à cette définition.

Une huile, au sens de la présente invention, est un composé lipophile, liquide à température ambiante, à changement d'état solide/liquide réversible.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arana, de tournesol, de ricin, d'avocat, les triglycérides des acides caprylique/caprique comme par exemple ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R⁶COOR⁷ et R⁶OR⁷ dans laquelle R⁶ représente une chaîne hydrocarbonée saturée ou insaturée (par exemple, le reste d'un acide gras) comportant de 8 à 29 atomes de carbone, et R⁷ représente une chaîne hydrocarbonée, ramifiée ou non, contenant de 3 à 30 atomes de carbone ; citons par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononaoate de diéthylèneglycol ; et les esters de pendaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné tel que l'huile de parléam ;
- les alcools gras fluides ayant de 8 à 26 atomes de carbone, comme par exemple l'octyldodécanol, le 2-butyloctanol, l'alcool oléique, l'alcool linoléique ou l'alcool linolénique ;
- les huiles fluorées partiellement hydrocarbonées comme celles décrites dans le document JP-A-2 295912. Comme huiles fluorées, on peut citer aussi le perfluorométhylcyclopentane et le perfluoro-1,3-diméthylcyclohexane, par exemple vendus sous les dénominations de « FLUTEC PC1® » et « FLUTEC PC3® » par la Société BNFL Fluorochemicals ; le perfluoro-1,2-diméthylcyclobutane ; les perfluoralcanes tels que le dodécafluoropentane et le tétradécafluorohexane, par exemple vendus sous les dénominations de « PF 5050® » et « PF 5060® » par la Société 3M, ou encore le bromoperfluorooctyle par exemple vendu sous la dénomination « FORALKYL® » par la Société Atochem ; le nanofluorométhoxybutane par exemple vendu sous la dénomination « MSX 4518® » par la Société 3M et le nanofluoroéthoxyisobutane ; les dérivés de perfluoromorpholine, tels que la 4-trifluorométhyl perfluoromorpholine par exemple vendue sous la dénomination « PF 5052® » par la Société 3M.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Une cire, au sens de la présente invention, est un composé lipophile, solide à température ambiante (environ 25 °C), à changement d'état solide/liquide réversible, ayant une température de fusion supérieure à environ 40 °C et pouvant aller jusqu'à 200 °C, et présentant à l'état solide une organisation cristalline anisotrope. Les cires animales et végétales comprennent comme constituants essentiels des esters d'acides carboxyliques et d'alcools à longues chaînes. D'une manière générale, la taille des cristaux de la cire est telle que les cristaux diffractent et/ou diffusent la lumière, conférant à la composition qui les comprend un aspect trouble plus ou moins opaque. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange, détectable microscopiquement et macroscopiquement (opalescence).

A titre de cires utilisables dans la présente invention, on peut citer les cires d'origine animale telles que la cire d'abeille, le spermaceti, la cire de lanoline et les dérivés de lanoline ; les cires végétales telles que les cires de tournesol, de riz, de pomme, la cire de Carnauba, de Candellila, d'Ouricury, du Japon, le beurre de cacao ou les cires de fibres de liège ou de canne à sucre ; les cires minérales, par exemple, de paraffine, de vaseline, de lignite ou les cires microcristallines, la cérésine ou l'ozokérite ; les cires synthétiques telles que les cires de polyéthylènes, les cires de Fischer-Tropsch ; les esters d'acides gras cireux ; les alcools gras cireux tels que par exemple les alcools myristylique, cétylique, stéarylique, arachidylique, béhénylique et érucylique , et leurs mélanges.

Le ou les corps gras non siliconés sont de préférence présents en une quantité allant de 0,1 à 90 % en poids, de préférence de 0,5 à 60 % en poids, et mieux encore de 1 à 40% en poids, par rapport au poids total de la composition.

Le ou les tensioactifs utilisables dans la composition selon l'invention peuvent être choisis parmi les tensioactifs cationiques, les tensioactifs anioniques, les tensioactifs non ioniques, les tensioactifs amphotères ou zwittérioniques et leurs mélanges.

La composition selon l'invention comprend de préférence au moins 0,01 % en poids de tensioactif(s), par rapport au poids total de la composition. De préférence, la composition selon l'invention contient de 0,05 à 20 % en poids de tensioactif(s), de manière plus préférée de 0,1 à 10 % en poids, et de manière encore plus préférée de 0,5 à 5% en poids, par rapport au poids total de la composition.

A titre d'exemple de tensioactifs anioniques utilisables dans les compositions selon la présente invention, on peut citer notamment (liste non limitative) les sels (en particulier les sels alcalins, notamment de sodium, les sels d'ammonium, les sels d'amines, les sels d'aminoalcools ou les sels d'alcalinoterreux (de magnésium)) des composés suivants : les alkylsulfates, les alkyléthersulfates, alkylamidoéthersulfates, alkylarylpolyéthersulfates, les monoglycérides sulfates ; les alkylsulfonates, les alkylphosphates, les alkylamidesulfonates, alkylarylsulfonates, les α-oléfine-sulfonates, lesparaffine-sulfonates ; les alkylsulfosuccinates ; les alkyléthersulfosuccinates, les alkylamidesulfosuccinates ; les alkylsulfosuccinamates ; les alkylsulfoacétates ; les alkylétherphosphates, les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle.

Parmi les tensioactifs anioniques utilisables, on peut également citer les sels d'acides gras tels que les sels acides oléique, ricinoléique, palmitique, stéarique ; les acides d'huile de coprah ou d'huile de coprah hydrogénée ; les acyl-lactylates dont le radical acyle comporte 8 à 20 atomes de carbone.

On peut également utiliser des tensioactifs faiblement anioniques, comme les acides d'alkyl D-galactoside uroniques et leurs sels ainsi que les acides alkyl éther carboxyliques, les acides alkyl (C₆ - C₂₄) éther carboxyliques polyoxyalkylénés, les acides alkyl (C₆-C₂₄)aryl éther carboxyliques polyoxyalkylénés, les acides alkyl (C₆-C₂₄) amino éther carboxyliques polyoxyalkylénés et les sels de ces acides, en particulier ceux comportant de 2 à 50 groupements oxyde d'éthylène et leurs mélanges.

Parmi les tensioactifs anioniques, on préfère utiliser selon l'invention les sels d'alkylsulfates, d'alkyléthersulfates, d'alkyléthercarboxylates ainsi que leurs mélanges.

Les tensioactifs cationiques utilisables dans les compositions de la présente invention comprennent par exemple les sels d'amines grasses primaires, secondaires ou tertiaires, éventuellement polyoxyalkylénées, les sels d'ammonium quaternaire, et leurs mélanges.

A titre de sels d'ammonium quaternaire, on peut notamment citer, par exemple :
- ceux répondant à la formule générale (I) suivante : 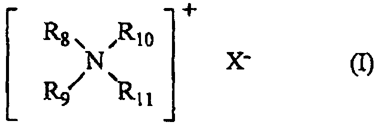 dans laquelle les radicaux R₈ à R₁₁, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont par exemple choisis parmi les radicaux alkyle en C₁₋₃₀, alcoxy en C₁₋₃₀, polyoxyalkylène (C₂-C₆), alkylamide en C₁₋₃₀, alkyl(C₁₂-C₂₂)amidoalkyle(C₂-C₆), alkyl(C₁₂-C₂₂)acétate, et hydroxyalkyle en C₁₋₃₀; X est un anion choisi dans le groupe des halogénures, phosphates, acétates, lactates, alkyl(C₂-C₆)sulfates, alkyl- ou alkylaryl-sulfonates.

Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier les chlorures de béhényltriméthylammonium, de distéaryldiméthylammonium, de cétyltriméthylammonium, de benzyldiméthylstéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.
- les sels d'ammonium quaternaire de l'imidazoline, comme par exemple ceux de formule (II) suivante : 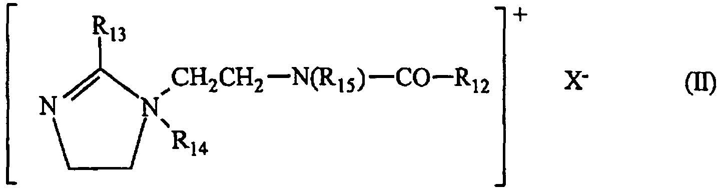 dans laquelle R₁₂ représente un radical alcényle ou alkyle comportant de 8 à 30 atomes de carbone, par exemple dérivés des acides gras du suif, R₁₃ représente un atome d'hydrogène, un radical alkyle en C₁-C₄ ou un radical alcényle ou alkyle comportant de 8 à 30 atomes de carbone, R₁₄ représente un radical alkyle en C₁-C₄, R₁₅ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₁₂ et R₁₃ désignent un mélange de radicaux alcényle ou alkyle comportant de 12 à 21 atomes de carbone, par exemple dérivés des acides gras du suif, R₁₄ désigne un radical méthyle, R₁₅ désigne un atome d'hydrogène. Un tel produit est par exemple commercialisé sous la dénomination REWOQUAT^{®} W 75 par la société REWO ;
- les sels de diammonium quaternaire de formule (III) : 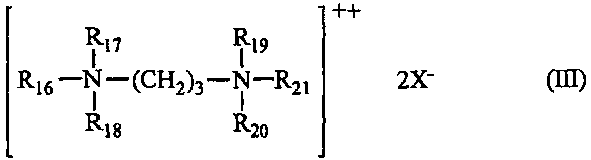 dans laquelle R₁₆ désigne un radical aliphatique comportant environ de 16 à 30 atomes de carbone ; R₁₇, R₁₈, R₁₉, R₂₀ et R₂₁, identiques ou différents sont choisis parmi un atome d'hydrogène et un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium ;
- les sels d'ammonium quaternaire contenant au moins une fonction ester, tels que ceux de formule (IV) suivante : 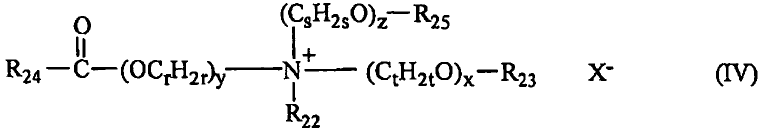 dans laquelle :
   R₂₂ est choisi parmi les radicaux alkyles en C₁-C₆ et les radicaux hydroxyalkyles ou dihydroxyalkyles en C₁-C₆ ;
   R₂₃ est choisi parmi :
      '- le radical 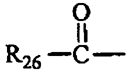
      - les radicaux R₂₇ hydrocarbonés en C₁-C₂₂, linéaires ou ramifiés, saturés ou insaturés,
      - l'atome d'hydrogène,
   R₂₅ est choisi parmi :
      - le radical 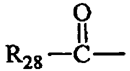
      - les radicaux R₂₉ hydrocarbonés en C₁-C₆, linéaires ou ramifiés, saturés ou insaturés,
      - l'atome d'hydrogène,
   R₂₄, R₂₆ et R₂₈, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
   sous réserve que la somme x + y + z vaut de 1 à 15, que lorsque x vaut 0 alors R₂₃ désigne R₂₇ et que lorsque z vaut 0 alors R₂₅ désigne R₂₉.

Les radicaux alkyles R₂₂ peuvent être linéaires ou ramifiés et plus particulièrement linéaires.

De préférence R₂₂ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₂₃ est un radical R₂₇ hydrocarboné, il peut être long et avoir de 12 à 22 atomes de carbone, ou court et avoir de 1 à 3 atomes de carbone.

Lorsque R₂₅ est un radical R₂₉ hydrocarboné, il a de préférence 1 à 3 atomes de carbone.

Avantageusement, R₂₄, R₂₆ et R₂₈, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₂₂ désigne un radical méthyle ou éthyle,
- x et y sont égaux à 1 ;
- z est égal à 0 ou 1 ;
- r, s et t sont égaux à 2 ;
- R₂₃ est choisi parmi :
   - le radical 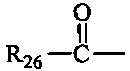
   - les radicaux méthyle, éthyle ou hydrocarbonés en C₁₄-C₂₂,
   - l'atome d'hydrogène ;
- R₂₅ est choisi parmi :
   - le radical 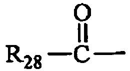
   - l'atome d'hydrogène ;
- R₂₄, R₂₆ et R₂₈, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyldiméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

La composition selon l'invention peut contenir par exemple un mélange de sels de mono-, di- et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Les tensioactifs non-ioniques utilisables dans les compositions de la présente invention sont des composés bien connus en soi (voir notamment à cet égard "Handbook of Surfactants" par M.R. PORTER, éditions Blackie & Son (Glasgow and London), 1991, pp 116-178). Ils sont choisis notamment parmi les alcools et alcools gras polyéthoxylés, polypropoxylés ou polyglycérolés, les alpha-diols polyéthoxylés, polypropoxylés ou polyglycérolés, les alkyl(C₁₋₂₀)phénols polyéthoxylés, polypropoxylés ou polyglycérolés, la chaîne grasse comportant, par exemple, de 8 à 18 atomes de carbone, le nombre de groupements oxyde d'éthylène ou oxyde de propylène pouvant aller notamment de 2 à 50 et le nombre de groupements glycérol pouvant aller notamment de 2 à 30.

On peut également citer les condensats d'oxyde d'éthylène et d'oxyde de propylène sur des alcools gras; les amides gras polyéthoxylés ayant de préférence de 2 à 30 motifs d'oxyde d'éthylène, les amides gras polyglycérolés comportant en moyenne de 1 à 5 groupements glycérol et en particulier de 1,5 à 4, les esters d'acides gras et de sorbitane éthoxylés ayant de 2 à 30 motifs d'oxyde d'éthylène, les esters d'acides gras du saccharose, les esters d'acides gras et de polyéthylèneglycol, les alkylpolyglycosides, les huiles végétales polyéthoxylées, les dérivés de N-(alkyl en C₆₋₂₄)glucamine, les oxydes d'amines tels que les oxydes d'(alkyl en C_{1.14})amines ou les oxydes de N-(acyl en C₁₀₋₁₄)-aminopropylmorpholine.

Les tensioactifs amphotères ou zwittérioniques utilisables dans les compositions de la présente invention comprennent par exemple les dérivés d'amines aliphatiques secondaires ou tertiaires, dans lesquels le groupe aliphatique est une chaîne linéaire ou ramifiée comportant de 8 à 22 atomes de carbone et contenant au moins un groupe anionique tel que, par exemple, un groupe carboxylate, sulfonate, sulfate, phosphate ou phosphonate. On peut citer également les alkyl(C₈₋₂₀)bétaïnes, les sulfobétaïnes, les (alkyl en C₈₋₂₀)amido(alkyl en C₆₋₈)bétaïnes ou les (alkyl en C₈₋₂₀)amido(alkyl en C₆₋₈)sulfobétaïnes.

Parmi les dérivés d'amines, on peut citer les produits commercialisés sous la dénomination MIRANOL^{®}, tels que décrits dans les brevets US 2 528 378 et US 2 781 354 et classés dans le dictionnaire CTFA, 3ème édition, 1982, sous les dénominations Amphocarboxyglycinate et Amphocarboxypropionate de structures respectives (1) et (2) :

Rₐ-CONHCH₂CH₂-N(R_{b})(R_{c})(CH₂COO⁻) (1)

dans laquelle :
Rₐ représente un groupe alkyle dérivé d'un acide Rₐ-COOH présent dans l'huile de coprah hydrolysée, un groupe heptyle, nonyle ou undécyle,
R_{b} représente un groupe bêta-hydroxyéthyle, et
R_{c} représente un groupe carboxyméthyle ;
et

Rₐ'-CONHCH₂CH₂-N(B)(B') (2)

dans laquelle :
B représente -CH₂CH₂OX',
B' représente -(CH₂)_{z}-Y', avec z = 1 ou 2,
X' représente le groupe -CH₂CH₂-COOH ou un atome d'hydrogène,
Y' représente -COOH ou le groupe -CH₂-CHOH-SO₃H,
Rₐ' représente un groupe alkyle d'un acide Rₐ'-COOH présent dans l'huile de coprah ou dans l'huile de lin hydrolysée, un groupe alkyle, notamment en C₁₇ et sa forme iso, un groupe en C₁₇ insaturé.

Ces composés sont classés dans le dictionnaire CTFA, 5ème édition, 1993, sous les dénominations cocoamphodiacétate de disodium, lauroamphodiacétate de disodium, caprylamphodiacétate de disodium, capryloamphodiacétate de disodium, cocoamphodipropionate de disodium, lauroamphodipropionate de disodium, caprylamphodipropionate de disodium, capryloamphodipropionate de disodium, acide lauroamphodipropionique, acide cocoamphodipropionique.

A titre d'exemple, on peut citer le cocoamphodiacétate commercialisé par la société RHODIA sous la dénomination commerciale MIRANOL^{®} C2M concentré.

Selon un premier mode de réalisation préféré, la composition selon l'invention comprend l'association d'un ou plusieurs alcools gras et d'un ou plusieurs tensioactifs cationiques.

Dans ce mode de réalisation, le ou les alcools gras comprennent de 8 à 40 atomes de carbone, et de préférence sont choisis parmi l'alcool cétylique, l'alcool stéarylique et leurs mélanges.

Le ou les tensioactifs cationiques sont les sels d'ammonium quaternaires tels que décrits ci-avant, ceux comportant au moins une chaîne béhényle étant particulièrement préférés.

Selon un second mode de réalisation également préféré, la composition selon l'invention comprend l'association de vaseline et/ou d'huile de vaseline avec un ou plusieurs tensioactifs non-ioniques tels que décrits ci-avant.

Dans ce mode de réalisation, le ou les tensioactifs non-ioniques préférés sont ceux comprenant au moins un groupement oxyéthylène, et de préférence polyoxyéthylène.

Les silicones employées dans les compositions selon l'invention peuvent se trouver sous forme soluble, dispersée ou micro-dispersée. La ou les silicones sont de préférence présentes en une quantité allant de 0,01 à 10% en poids, et plus préférentiellement de 0,1 à 5% en poids, par rapport au poids total de la composition.

A titre d'exemple de silicones préférées, on peut notamment citer les huiles siliconées, telles que par exemple les polydiméthylsiloxanes linéaires ou cycliques, et les silicones aminées telles que les amodiméthicones.

Les compositions selon l'invention peuvent également contenir un ou plusieurs agents épaississants, qui peuvent être choisis parmi les épaississants polymériques naturels ou synthétiques, anioniques, amphotères, zwittérioniques, non ioniques ou cationiques, associatifs ou non, et les épaississants non polymériques tels que par exemple un électrolyte ou un sucre.

Comme agents épaississants polymériques, citons par exemple les agents épaississants cellulosiques, par exemple l'hydroxyéthylcellulose, l'hydroxypropylcellulose et le carboxyméthylcellulose, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, les agents épaississants polymériques synthétiques tels que les homopolymères réticulés d'acide acrylique ou d'acide acrylamidopropanesulfonique, par exemple le Carbomer, les polymères associatifs non ioniques, anioniques ou amphotères, tels que les polymères commercialisés sous les appellations PEMULEN TR1 ou TR2 par la société GOODRICH, SALCARE SC90 par la société ALLIED COLLOIDS, ACULYN 22, 28, 33, 44 ou 46 par la société ROHM & HAAS et ELFACOS T210 et T212 par la société AKZO.

Les compositions conformes à l'invention peuvent être conditionnées par exemple dans un pot, dans un tube, un flacon pompe, ou dans un dispositif aérosol usuel en cosmétique.

Les compositions selon l'invention peuvent, lorsqu'elles ont destinées à être conditionnées dans un dispositif de type aérosol, contenir un ou plusieurs gaz propulseurs.

Le gaz propulseur peut alors être choisi par exemple parmi le diméthyléther, les alcanes en C₃ à C₅, les hydrocarbures halogénés, et leurs mélanges.

Les compositions selon l'invention peuvent en outre contenir un ou plusieurs additifs choisis parmi les agents nacrants ; les agents opacifiants ; les agents plastifiants ; les filtres solaires ; les parfums ; les colorants ; les conservateurs ; les agents de stabilisation du pH ; les acides ; les bases ; les polyols (par exemple les glycols) ; les charges minérales ; les paillettes, et tout autre additif classiquement utilisé dans le domaine cosmétique.

L'homme de métier veillera à choisir les éventuels additifs et leurs quantités de manière à ce qu'ils ne nuisent pas aux propriétés des compositions de la présente invention.

Ces additifs peuvent être présents dans la composition selon l'invention en une quantité allant de 0 à 50 % en poids par rapport au poids total de la composition.

Les compositions selon l'invention peuvent se présenter entre autres sous forme de liquides plus ou moins épaissis, de gels, de crèmes, de pâtes ou de mousses.

La composition selon l'invention peut être avantageusement utilisée pour le traitement cosmétique des cheveux.

Elle peut également être utilisée pour le soin des cheveux, en particulier sous forme de soin non-rincé.

Selon un mode de réalisation particulièrement préféré, la composition selon l'invention est utilisée pour le coiffage des cheveux, et plus préférentiellement pour le coiffage et le conditionnement simultanés des cheveux.

La présente invention concerne également un procédé de traitement cosmétique des cheveux, par exemple un procédé de soin capillaire, ou un procédé de mise en forme et/ou de maintien de la coiffure, qui consiste à appliquer sur les cheveux une quantité efficace d'une composition telle que décrite ci-dessus, puis à effectuer un éventuel rinçage après un éventuel temps de pose.

De préférence, la composition selon l'invention n'est pas rincée.

La présente invention a enfin pour objet l'utilisation, pour la mise en forme et/ou la fixation de la coiffure, d'une composition cosmétique comprenant, dans un milieu aqueux cosmétiquement acceptable :
- un ou plusieurs corps gras non siliconés,
- un ou plusieurs tensioactifs, et
- un ou plusieurs copolymères vinylformamide / vinylamine comprenant :
   de 10 à 95 % en moles de motifs de formule suivante A : 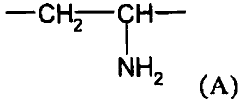
   et de 90 à 5 % en moles de motifs de formule suivante B:

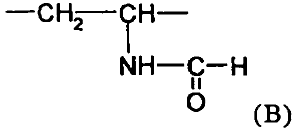

Cette utilisation s'est avérée particulièrement efficace pour diminuer le volume de la chevelure et/ou plaquer les cheveux.

Les exemples suivants sont donnés à titre illustratif de la présente invention.

### EXEMPLES

Dans les exemples suivants, toutes les quantités sont indiquées en pour cent en poids de matière active (MA) par rapport au poids total de la composition, sauf indication contraire.

### Exemple 1

Cet exemple illustre la formulation d'une crème de coiffage conforme à l'invention.

Une telle crème a été préparée à partir des ingrédients indiqués dans le tableau ci-dessous :

| Ingrédients | Teneurs |
|---|---|
| Copolymère vinvlformamide / vinylamine (1) | 2 % |
| Alcool cétylstéarylique | 5 % |
| Mélange myristate/palmitate/stearate de myristyle/cétyle/stéarvle (2) | 1 % |
| Chlorure de béhényltriméthylammonium (3) | 0,8 % |
| Amodiméthicone (4) | 1,5 % |
| Eau déminéralisée | qsp 100% |

| | |
|---|---|
| (1) commercialisé sous la dénomination LUPAMIN 9030 par la société BASF. (2) commercialisé sous la dénomination MIRACET par la société LASERSON. (3) commercialisé sous la dénomination GENAMIN KDMP par la société CLARIANT. (4) commercialisé sous la dénomination DC 939 EMULSION par la société DOW CORNING. | |

Cette crème s'est avérée particulièrement appropriée pour le coiffage et le soin des cheveux frisés.

L'application de cette crème sur cheveux frisés a en effet permis d'obtenir des effets de diminution de volume, et une bonne définition des boucles avec une tenue durable. En outre, le toucher des cheveux après shampooing s'est avéré particulièrement doux et agréable.

### Exemple 2 :

Cet exemple illustre la formulation d'une émulsion de coiffage conforme à l'invention.

Une telle émulsion a été préparée à partir des ingrédients indiqués dans le tableau ci-dessous ;

| Ingrédients | Teneurs |
|---|---|
| Copolymère vinylformamide / vinvlamine (1) | 6 % |
| Huile de vaseline (5) | 36,7 % |
| Vaseline blanche (6) | 4 % |
| Polydiméthylsiloxane en émulsion aqueuse dans un mélange de lauryl-éthers polyoxyéthylène (7) | 0,8 % |
| Glycérol | 4 % |
| Mono-laurate de sorbitan polyoxyéthyléné (8) | 2 % |
| Alcool cétylstéarylique | 2 % |
| Mélange de mono/distéarate de gylcéryle et de stéarate de polyéthylèneglycol (100 OE) (9) | 4,5 % |
| Eau déminéralisée | qsp 100% |

| | |
|---|---|
| (1) commercialisé sous la dénomination LUPAMIN 9030 par la société BASF. (5) commercialisée sous la dénomination MARCOL 82 par la société EXXONMOBIL. (6) commercialisée sous la dénomination CODEX 236 par la société AIGLON. (7) commercialisé sous la dénomination DC 1664 EMULSION par la société DOW CORNING. (8) commercialisée sous la dénomination TWEEN 20 par la société UNIQEMA. (9) commercialisée sous la dénomination ARLACEL 165 FL par la société UNIQEMA. | |

Cette émulsion s'est avérée particulièrement appropriée pour le coiffage et le soin des cheveux ethniques.

L'application de cette composition sur cheveux africains défrisés a en effet permis d'obtenir des coiffures plaquées longue tenue (tenue de plusieurs jours).

Sur cheveux très frisés et secs de type cheveux brésiliens, l'application de cette composition a permis d'obtenir un très bon contrôle du volume, et une belle définition de la frisure. En outre, le toucher des cheveux après le shampooing s'est avéré particulièrement doux.

### Exemple 3 comparatif :

On a réalisé les deux compositions suivantes :

| | Composition 1 (invention) | Composition 2 (hors invention) |
|---|---|---|
| Polyvinylformamide hydrolysée à 30% (1) | 6% | - |
| N-vinylformamide (2) | - | 6% |
| Huile de vaseline (3) | 36.7% | 36.7% |
| Vaseline blanche (4) | 4% | 4% |
| Polydimethyl siloxane en émulsion aqueuse dans mélange lauryl ethers polyoxyethylenes/eau (5) | 0.8% | 0.8% |
| Glycérol | 4% | 4% |
| Mono-laurate de sorbitane oxyethyléné (6) | 2% | 2% |
| Alcool cétylstéarylique (7) | 2% | 2% |
| Mélange mono/distearate de glyceryle / stearate de polyethylene glycol (100 OE) (8) | 4.5% | 4.5% |
| Eau déminéralisée | Qsp100 | Qsp100 |

| | | |
|---|---|---|
| (1) commercialisé sous la dénomination LUPAMIN 9030 par la société BASF (2) commercialisé sous la dénomination LUPAMIN 9000 par la société BASF (3) commercialisé sous la dénomination MARCOL 82 par la société EXXONMOBIL (4) commercialisé sous la dénomination CODEX 236 par la société AIGLON (5) commercialisé sous la dénomination DC 1664 EMULSION par la société DOW CORNING (6) commercialisé sous la dénomination TWEEN 20 par la société UNIQEMA (7) commercialisé sous la dénomination ECOROL 68/50 P par la société ECOGREEN OLEOCHEMICALS (8) commercialisé sous la dénomination ARLACEL 165 FL par la société UNIQEMA | | |

Les deux compositions précédentes ont été appliquées, à raison de 1g de formulation par mèche, sur des mèches de cheveux châtains humides de 2.7g préalablement shampouinées, rincées et essorées.

En final, les mèches non rincées ont ensuite été séchées sous casque pendant 30 minutes, puis démêlées.

Un panel de 7 testeurs a évalué la cohésion du méché obtenu avec les compositions 1 et 2 en les classant suivant ce critère : rang 1 pour la mèche avec les cheveux présentant le méché le plus cohésif, rang 2 pour la mèche avec les cheveux présentant le méché le moins cohésif.

Les 7 évaluateurs ont trouvé un méché plus cohésif avec la composition 1 selon l'invention.

La somme des rangs obtenue pour chaque composition est :7 pour la composition 1, 14 pour la composition 2.

Selon le test de KRAMER (A non parametric ranking method for the statistical evaluation of sensory data, chemical Senses and Flavor (1974)121-123), deux compositions sont significativement différentes au seuil de 5% si la somme des rangs se situe en dehors de l'intervalle de 8 à 13.

7 étant inférieur à l'intervalle 8-13, la cohésion du méché est significativement plus importante avec la composition 1 selon l'invention.

## Revendications

1. Composition cosmétique comprenant, dans un milieu aqueux cosmétiquement acceptable :
- un ou plusieurs corps gras non siliconés,
- un ou plusieurs tensioactifs,
- un ou plusieurs copolymères vinylformamide / vinylamine comprenant :
de 20 à 40 % en moles de motifs de formule suivante A : 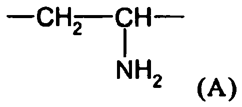
et de 60 à 80 % en moles de motifs de formule suivante B: 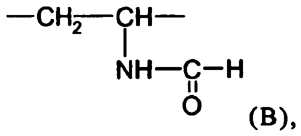 et
- une ou plusieurs silicones.

2. Composition selon la revendication 1, **caractérisée en ce que** le ou les copolymères vinylformamide / vinylamine sont constitués uniquement de motifs de formule A et de motifs de formule B.

3. Composition selon l'une quelconque des revendications précédentes, **caractérisée en ce que** le ou les copolymères vinylformamide / vinylamine sont présents dans des proportions allant de 0,05 à 25 % en poids, et de préférence de 0,1 à 20 % en poids, par rapport au poids total de la composition.

4. Composition selon l'une quelconque des revendications précédentes, **caractérisée en ce que** le ou les corps gras non siliconés sont choisis parmi les huiles, les cires, les résines non siliconées organiques ou minérales, naturelles ou synthétiques.

5. Composition selon la revendication précédente, **caractérisée en ce qu'**elle comprend une ou plusieurs huiles choisies parmi :
- les huiles hydrocarbonées d'origine animale ;
- les huiles hydrocarbonées d'origine végétale ;
- les esters et les éthers de synthèse, notamment d'acides gras, comme les huiles de formules R⁶COOR⁷ et R⁶OR⁷ dans laquelle R⁶ représente une chaîne hydrocarbonée saturée ou insaturée comportant de 8 à 29 atomes de carbone, et R⁷ représente une chaîne hydrocarbonée, ramifiée ou non, contenant de 3 à 30 atomes de carbone ;
- les hydrocarbures linéaires ou ramifiés, d'origine minérale ou synthétique, tels que les huiles de paraffine, volatiles ou non, et leurs dérivés, la vaseline, l'huile de vaseline, les polydécènes, le polyisobutène hydrogéné;
- les alcools gras fluides ayant de 8 à 26 atomes de carbone;
- les huiles fluorées partiellement hydrocarbonées ;
et/ou une ou plusieurs cires choisies parmi les cires d'origine animale; les cires végétales; les cires minérales; les cires synthétiques les esters d'acides gras cireux ; les alcools gras cireux et leurs mélanges.

6. Composition selon l'une quelconque des revendications précédentes, **caractérisée en ce que** le ou les corps gras non siliconés sont présents en une quantité allant de 0,1 à 90 % en poids, de préférence de 0,5 à 60 % en poids, et de manière plus préférée de 1 à 40% en poids, par rapport au poids total de la composition.

7. Composition selon l'une quelconque des revendications précédentes, **caractérisée en ce que** le ou les tensioactifs sont choisis parmi les tensioactifs cationiques, les tensioactifs anioniques, les tensioactifs non ioniques, les tensioactifs amphotères ou zwittérioniques, et leurs mélanges.

8. Composition selon la revendication 7, **caractérisée en ce qu'**elle comprend un ou plusieurs tensioactifs cationiques choisis parmi les sels d'amines grasses primaires, secondaires ou tertiaires, éventuellement polyoxyalkylénées, les sels d'ammonium quaternaire, et leurs mélanges.

9. Composition selon la revendication 7, **caractérisée en ce qu'**elle comprend un ou plusieurs tensioactifs non-ioniques choisis parmi :
- les alcools et alcools gras polyéthoxylés, polypropoxylés ou polyglycérolés, les alpha-diols polyéthoxylés, polypropoxylés ou polyglycérolés, les alkyl(C₁₋₂₀)phénols polyéthoxylés, polypropoxylés ou polyglycérolés, la chaîne grasse comportant de 8 à 18 atomes de carbone, le nombre de groupements oxyde d'éthylène ou oxyde de propylène pouvant aller de 2 à 50 et le nombre de groupements glycérol pouvant aller de 2 à 30 ;
- les condensats d'oxyde d'éthylène et d'oxyde de propylène sur des alcools gras; les amides gras polyéthoxylés ayant de 2 à 30 motifs d'oxyde d'éthylène, les amides gras polyglycérolés comportant en moyenne de 1 à 5 groupements glycérol, les esters d'acides gras et de sorbitane éthoxylés ayant de 2 à 30 motifs d'oxyde d'éthylène, les esters d'acides gras du saccharose, les esters d'acides gras et de polyéthylèneglycol, les alkylpolyglycosides, les huiles végétales polyéthoxylées, les dérivés de N-(alkyl en C₆₋₂₄)glucamine, les oxydes d'amines tels que les oxydes d'(alkyl en C₁₀₋₁₄)amines ou les oxydes de N-(acyl en C₁₀₋₁₄)-aminopropylmorpholine.

10. Composition selon l'une quelconque des revendications précédentes, **caractérisée en ce qu'**elle comprend au moins 0,01 % en poids de tensioactif(s), et de préférence de 0,05 à 20 % en poids, par rapport au poids total de la composition.

11. Composition selon la revendication 8, **caractérisée en ce qu'**elle comprend l'association d'un ou plusieurs alcools gras comprenant de 8 à 40 atomes de carbone et d'un ou plusieurs tensioactifs cationiques choisis parmi les sels d'ammonium quaternaires.

12. Composition selon la revendication 9, **caractérisée en ce qu'**elle comprend l'association de vaseline et/ou d'huile de vaseline avec un ou plusieurs tensioactifs non-ioniques.

13. Procédé de traitement cosmétique des cheveux, **caractérisé en ce qu'**il consiste à appliquer sur les cheveux une quantité efficace d'une composition selon l'une quelconque des revendications précédentes, puis à effectuer un éventuel rinçage après un éventuel temps de pose.

14. Utilisation, pour la mise en forme et/ou la fixation de la coiffure, d'une composition cosmétique comprenant, dans un milieu aqueux cosmétiquement acceptable :
- un ou plusieurs corps gras non siliconés,
- un ou plusieurs tensioactifs, et
- un ou plusieurs copolymères vinylformamide / vinylamine comprenant :
de 10 à 95 % en moles de motifs de formule suivante A : 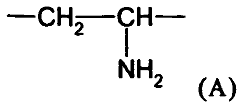
et de 90 à 5 % en moles de motifs de formule suivante B:

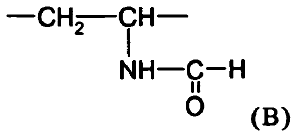

15. Utilisation d'une composition selon l'une quelconque des revendications 1 à 12, pour le coiffage et le conditionnement simultanés des cheveux.

## Patentansprüche

1. Kosmetische Zusammensetzung, die in einem kosmetisch unbedenklichen wässrigen Medium:
- eine oder mehrere Nichtsilikon-Fettsubstanzen,
- ein oder mehrere Tenside,
- ein oder mehrere Vinylformamid/Vinylamin-Copolymere, umfassend:
20 bis 40 Mol-% Einheiten der folgenden Formel A: 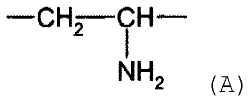
und 60 bis 80 Mol-% Einheiten der folgenden Formel B: 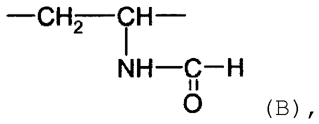 und
- ein oder mehrere Silikone
umfasst.

2. Zusammensetzung nach Anspruch 1, **dadurch gekennzeichnet, dass** das Vinylformamid/Vinylamin-Copolymer bzw. die Vinylformamid/Vinylamin-Copolymere ausschließlich aus Einheiten der Formel A und Einheiten der Formel B aufgebaut ist bzw. sind.

3. Zusammensetzung nach einem der vorhergehenden Ansprüche, **dadurch gekennzeichnet, dass** das Vinylformamid/Vinylamin-Copolymer bzw. die Vinylformamid/Vinylamin-Copolymere in Anteilen im Bereich von 0,05 bis 25 Gew.-% und vorzugsweise 0,1 bis 20 Gew.-%, bezogen auf das Gesamtgewicht der Zusammensetzung, vorliegt bzw. vorliegen.

4. Zusammensetzung nach einem der vorhergehenden Ansprüche, **dadurch gekennzeichnet, dass** die Nichtsilikon-Fettsubstanz bzw. die Nichtsilikon-Fettsubstanzen aus Ölen, Wachsen, natürlichen oder synthetischen, organischen oder anorganischen Nichtsilikon-Wachsen ausgewählt ist bzw. sind.

5. Zusammensetzung nach dem vorhergehenden Anspruch, **dadurch gekennzeichnet, dass** sie ein oder mehrere Öle, ausgewählt aus
- Kohlenwasserstoffölen tierischen Ursprungs;
- Kohlenwasserstoffölen pflanzlichen Ursprungs;
- synthetischen Estern und Ethern, insbesondere von Fettsäuren, wie Ölen der Formeln R⁶COOR⁷ und R⁶OR⁷, worin R⁶ für eine gesättigte oder ungesättigte Kohlenwasserstoffkette mit 8 bis 29 Kohlenstoffatomen steht und R⁷ für eine verzweigte oder unverzweigte Kohlenwasserstoffkette mit 3 bis 30 Kohlenstoffatomen steht;
- linearen oder verzweigten Kohlenwasserstoffen mineralischen oder synthetischen Ursprungs, wie flüchtigen oder nichtflüchtigen Paraffinölen und Derivaten davon, Vaseline, Vaselineöl, Polydecenen oder hydriertem Polyisobuten;
- fließfähigen Fettalkoholen mit 8 bis 26 Kohlenstoffatomen;
- teilfluorierten Kohlenwasserstoffölen;
und/oder ein oder mehrere Wachse, ausgewählt aus Wachsen tierischen Ursprungs; pflanzlichen Wachsen; mineralischen Wachsen; synthetischen Wachsen; wachsartigen Fettsäureestern; wachsartigen Fettalkoholen und Mischungen davon; umfasst.

6. Zusammensetzung nach einem der vorhergehenden Ansprüche, **dadurch gekennzeichnet, dass** die Nichtsilikon-Fettsubstanz bzw. die Nichtsilikon-Fettsubstanzen in einer Menge im Bereich von 0,1 bis 90 Gew.-%, vorzugsweise 0,5 bis 60 Gew.-% und weiter bevorzugt 1 bis 40 Gew.-%, bezogen auf das Gesamtgewicht der Zusammensetzung, vorliegt bzw. vorliegen.

7. Zusammensetzung nach einem der vorhergehenden Ansprüche, **dadurch gekennzeichnet, dass** das Tensid bzw. die Tenside aus kationischen Tensiden, anionischen Tensiden, nichtionischen Tensiden, amphoteren oder zwitterionischen Tensiden und Mischungen davon ausgewählt ist bzw. sind.

8. Zusammensetzung nach Anspruch 7, **dadurch gekennzeichnet, dass** sie ein oder mehrere kationische Tenside, die aus gegebenenfalls polyoxyalkylenierten primären, sekundären oder tertiären Fettaminen, quartären Ammoniumsalzen und Mischungen davon ausgewählt sind, umfasst.

9. Zusammensetzung nach Anspruch 7, **dadurch gekennzeichnet, dass** sie ein oder mehrere nichtionische Tenside, die aus:
- polyethoxylierten, polypropoxylierten oder polyglycerinierten Alkoholen und Fettalkoholen, polyethoxylierten, polypropoxylierten oder polyglycerinierten alpha-Diolen, polyethoxylierten, polypropoxylierten oder polyglycerinierten (C₁₋₂₀)Alkylphenolen, wobei die Fettkette 8 bis 18 Kohlenstoffatome enthält, die Zahl der Ethylenoxid- bzw. Propylenoxidgruppen im Bereich von 2 bis 50 liegen kann und die Zahl der Glyceringruppen im Bereich von 2 bis 30 liegen kann;
- Kondensaten von Ethylenoxid und Propylenoxid mit Fettalkoholen; polyethoxylierten Fettamiden mit 2 bis 30 Ethylenoxideinheiten, polyglycerinierten Fettamiden mit durchschnittlich 1 bis 5 Glycerineinheiten, ethoxylierten Estern von Fettsäuren und Sorbitan mit 2 bis 30 Ethylenoxideinheiten, Saccharosefettsäureestern, Ester von Fettsäuren und Polyethylenglykol, Alkylpolyglycosiden, polyethoxylierten pflanzlichen Ölen, N-(C₆₋₂₄₋Alkyl)-glucaminderivaten, Aminoxiden wie (C₁₀₋₁₄-Alkyl)-aminoxiden oder N- (C₁₀₋₁₄-Acyl) aminopropylmorpholinoxiden;
ausgewählt sind, umfasst.

10. Zusammensetzung nach einem der vorhergehenden Ansprüche, **dadurch gekennzeichnet, dass** sie mindestens 0,01 Gew.-% Tensid(e) und vorzugsweise 0,05 bis 20 Gew.-%, bezogen auf das Gesamtgewicht der Zusammensetzung, umfasst.

11. Zusammensetzung nach Anspruch 8, **dadurch gekennzeichnet, dass** sie die Kombination eines oder mehrerer Fettalkohole mit 8 bis 40 Kohlenstoffatomen und eines oder mehrerer kationischer Tenside, die aus quartären Ammoniumsalzen ausgewählt sind, umfasst.

12. Zusammensetzung nach Anspruch 9, **dadurch gekennzeichnet, dass** sie die Kombination von Vaseline und/oder Vaselineöl mit einem oder mehreren nichtionischen Tensiden umfasst.

13. Verfahren zur kosmetischen Behandlung der Haare, **dadurch gekennzeichnet, dass** es darin besteht, dass man auf die Haare eine wirksame Menge einer Zusammensetzung nach einem der vorhergehenden Ansprüche aufbringt und dann nach einer fakultativen Einwirkungszeit eine fakultative Spülung durchführt.

14. Verwendung einer kosmetischen Zusammensetzung, die in einem kosmetisch unbedenklichen wässrigen Medium:
- eine oder mehrere Nichtsilikon-Fettsubstanzen,
- ein oder mehrere Tenside und
- ein oder mehrere Vinylformamid/VinylaminCopolymere, umfassend:
10 bis 95 Mol-% Einheiten der folgenden Formel A: 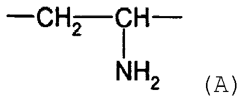
und 90 bis 5 Mol-% Einheiten der folgenden Formel B: 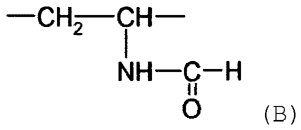
umfasst, zum Gestalten und/oder Fixieren der Frisur.

15. Verwendung einer Zusammensetzung nach einem der Ansprüche 1 bis 12 zum gleichzeitigen Frisieren und Konditionieren der Haare.

## Claims

1. Cosmetic composition comprising, in a cosmetically acceptable aqueous medium:
- one or more non-silicone fatty substances,
- one or more surfactants,
- one or more vinylformamide/vinylamine copolymers comprising:
from 20 to 40 mol% of units of following formula A: 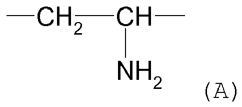
and from 60 to 80 mol% of units of following formula B: 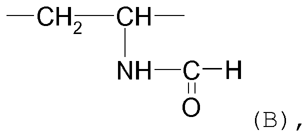 and
- one or more silicones.

2. Composition according to Claim 1, **characterized in that** the vinylformamide/vinylamine copolymer or copolymers are composed solely of units of formula A and of units of formula B.

3. Composition according to either one of the preceding claims, **characterized in that** the vinylformamide/vinylamine copolymer or copolymers are present in proportions ranging from 0.05 to 25% by weight and preferably from 0.1 to 20% by weight, with respect to the total weight of the composition.

4. Composition according to any one of the preceding claims, **characterized in that** the non-silicone fatty substance or substances are chosen from organic or inorganic and natural or synthetic non-silicone oils, waxes or resins.

5. Composition according to the preceding claim, **characterized in that** it comprises one or more oils chosen from:
- hydrocarbon oils of animal origin;
- hydrocarbon oils of vegetable origin;
- synthetic esters and ethers, in particular of fatty acids, such as oils of formulae R⁶COOR⁷ and _{R}⁶_{0R}⁷ in which R⁶ represents a saturated or unsaturated hydrocarbon chain comprising from 8 to 29 carbon atoms and R⁷ represents a branched or unbranched hydrocarbon chain comprising from 3 to 30 carbon atoms;
- linear or branched hydrocarbons of mineral or synthetic origin, such as liquid paraffins, which may or may not be volatile, and their derivatives, petrolatum, liquid petrolatum, polydecenes or hydrogenated polyisobutene;
- fluid fatty alcohols having from 8 to 26 carbon atoms;
- fluorinated oils which partially comprise hydrocarbon;
and/or one or more waxes chosen from waxes of animal origin; vegetable waxes; mineral waxes; synthetic waxes; waxy fatty acid esters; waxy fatty alcohols and their mixtures.

6. Composition according to any one of the preceding claims, **characterized in that** the non-silicone fatty substance or substances are present in an amount ranging from 0.1 to 90% by weight, preferably from 0.5 to 60% by weight and more preferably from 1 to 40% by weight, with respect to the total weight of the composition.

7. Composition according to any one of the preceding claims, **characterized in that** the surfactant or surfactants are chosen from cationic surfactants, anionic surfactants, nonionic surfactants, amphoteric or zwitterionic surfactants and their mixtures.

8. Composition according to Claim 7, **characterized in that** it comprises one or more cationic surfactants chosen from salts of optionally polyoxyalkylenated primary, secondary or tertiary fatty amines, quaternary ammonium salts and their mixtures.

9. Composition according to Claim 7, **characterized in that** it comprises one or more nonionic surfactants chosen from:
- polyethoxylated, polypropoxylated or polyglycerolated alcohols and fatty alcohols, polyethoxylated, polypropoxylated or polyglycerolated α-diols, or polyethoxylated, polypropoxylated or polyglycerolated (C₁₋₂₀)alkylphenols, the fatty chain comprising from 8 to 18 carbon atoms, it being possible for the number of ethylene oxide or propylene oxide groups to range from 2 to 50 and for the number of glycerol groups to range from 2 to 30;
- condensates of ethylene oxide and of propylene oxide with fatty alcohols; polyethoxylated fatty amides having from 2 to 30 ethylene oxide units, polyglycerolated fatty amides comprising on average from 1 to 5 glycerol groups, ethoxylated esters of fatty acids and of sorbitan having from 2 to 30 ethylene oxide units, sucrose fatty acid esters, esters of fatty acids and of polyethylene glycol, alkylpolyglycosides, polyethoxylated vegetable oils, N-(C₆₋₂₄ alkyl)glucamine derivatives or amine oxides, such as (C₁₀₋₁₄ alkyl) amine oxides or N-(C₁₀₋₁₄ acyl) aminopropylmorpholine oxides.

10. Composition according to any one of the preceding claims, **characterized in that** it comprises at least 0.01% by weight of surfactant(s), preferably from 0.05 to 20% by weight, with respect to the total weight of the composition.

11. Composition according to Claim 8, **characterized in that** it comprises the combination of one or more fatty alcohols comprising from 8 to 40 carbon atoms and of one or more cationic surfactants chosen from quaternary ammonium salts.

12. Composition according to Claim 9, **characterized in that** it comprises the combination of petrolatum and/or of liquid petrolatum with one or more nonionic surfactants.

13. Method for the cosmetic treatment of the hair, **characterized in that** it consists in applying, to the hair, an effective amount of a composition according to any one of the preceding claims and in then carrying out an optional rinsing after an optional setting time.

14. Use for the shaping and/or fixing of the hairstyle, of a cosmetic composition comprising, in a cosmetically acceptable aqueous medium:
- one or more non-silicone fatty substances,
- one or more surfactants, and
- one or more vinylformamide/vinylamine copolymers comprising:
from 10 to 95 mol% of units of following formula A: 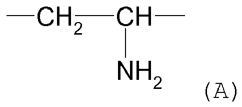
and from 90 to 5 mol% of units of following formula B:

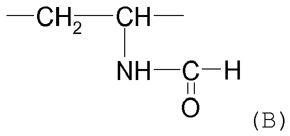

15. Use of a composition according to any one of Claims 1 to 12 for the simultaneous styling and conditioning of the hair.
